# EUROPEAN PATENT SPECIFICATION

(11) **EP 0 773 216 B1**
(45) Date of publication and mention of the grant of the patent: **04.12.2002**
(21) Application number: 96308020.5
(22) Date of filing: 05.11.1996
(51) Int. Cl.: C07D 311/74, C07C 69/75

(54) **Process for the production of chroman carboxylates**
Verfahren zur Herstellung von Chromancarbonsäureestern
Procédé de fabrication d'esters d'acide chromannecarboxyliques

(30) Priority: 09.11.1995 US 6487
(43) Date of publication of application: 14.05.1997
(73) Proprietor: Dow AgroSciences LLC, Indianapolis, Indiana 46268 (US)
(72) Inventor: Hormann, Robert Eugene, Philadelphia, Pennsylvania 19119 (US)
(74) Representative: Dey, Michael, Dr.

(56) References cited:
- DATABASE WPI Week 9529 Derwent Publications Ltd., London, GB; AN 95-220760 XP002021539 & JP-A-07 133 245 (NIPPON KAYAKU K. K.) , 23 May 1995
- DATABASE WPI Week 9512 Derwent Publications Ltd., London, GB; AN 95-085399 XP002021540 & JP-A-07 010 866 (NIPPON KAYAKU) , 13 January 1995
- CHEMICAL ABSTRACTS, vol. 69, no. 7, 12 August 1968 Columbus, Ohio, US; abstract no. 26830v, G. SARKAR, D. NASIPURI: "Cyclohexenone derivatives. V. Further studies on alkylation of Hagemann's ester" page 2487; XP002021538 & J. INDIAN. CHEM. SOC., vol. 45, no. 3, 1968, pages 200-204,

## Description

This invention relates to an improved process for the preparation of 5-methylchroman-6-carboxylic acid, a useful intermediate in the preparation of chroman-containing diacylhydrazines which are useful as insecticides.

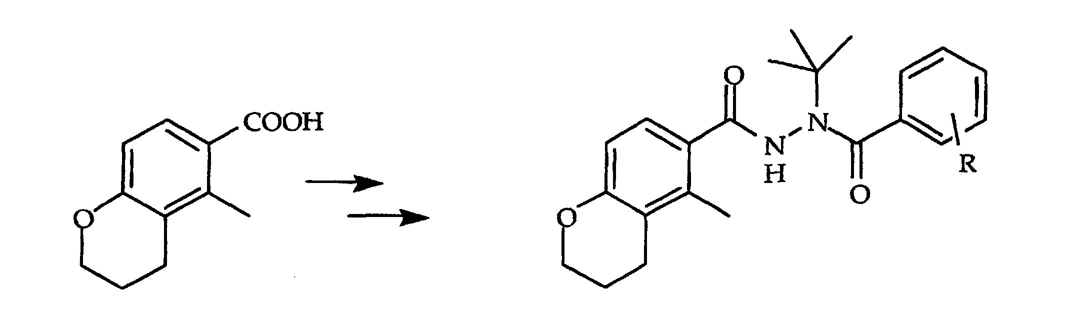

Published procedures, such as those disclosed in JP 7-010866, JP 7-109267, JP 7-109269, JP 7-126263, JP 7-133245, JP 7-133270, JP 7-133271 and JP 7-179452, are inconvenient, lengthy or poor-yielding. The process of the present invention is shorter or more convenient than those previously described and thus leads to a practical procedure for the eventual manufacture of the final N-chromanoyl-N'-*tert*-butyl-N'-benzoylhydrazine type insecticides which can be consequently offered to the marketplace in a more economical fashion.

The process of the present invention comprises the steps of
a. reacting a Hagemann's ester (**1**) with a propargyl derivative (**2**) in the presence of a base and a solvent to produce a propargyl ether (**3**) 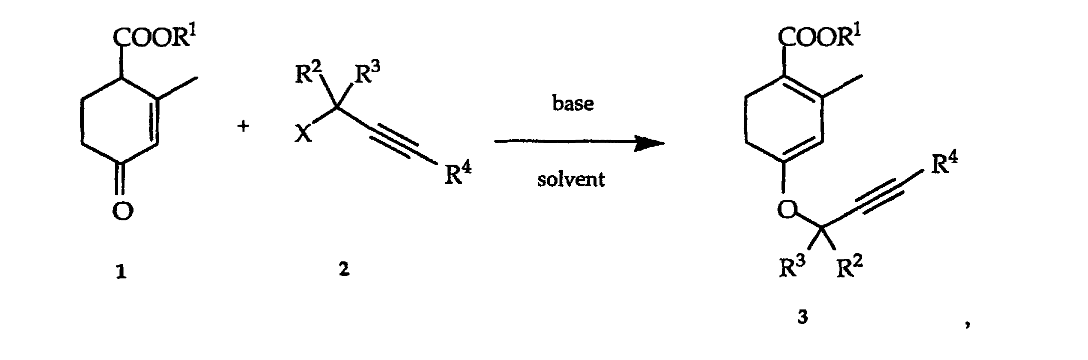 Followed by either:
b1. rearranging the propargyl ether (**3**) by application of heat, optionally with a suitable catalyst and a solvent being present, to produce a cyclic ether type compound (**4**) 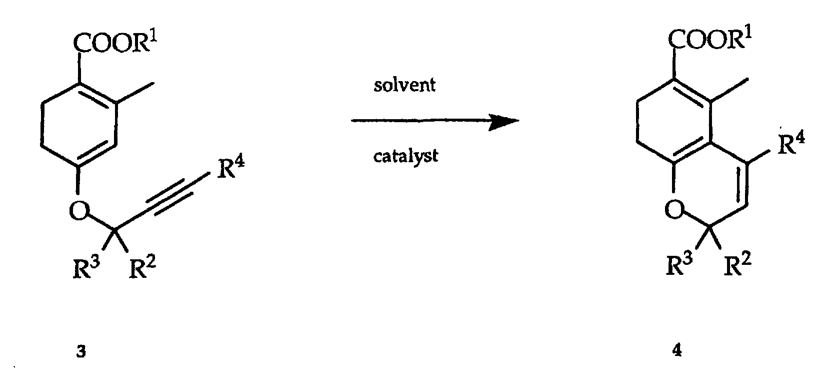 and
c1. isomerizing the cyclic ether type compound (**4**) with a catalyst in a suitable solvent to produce a chroman ester (**5**) 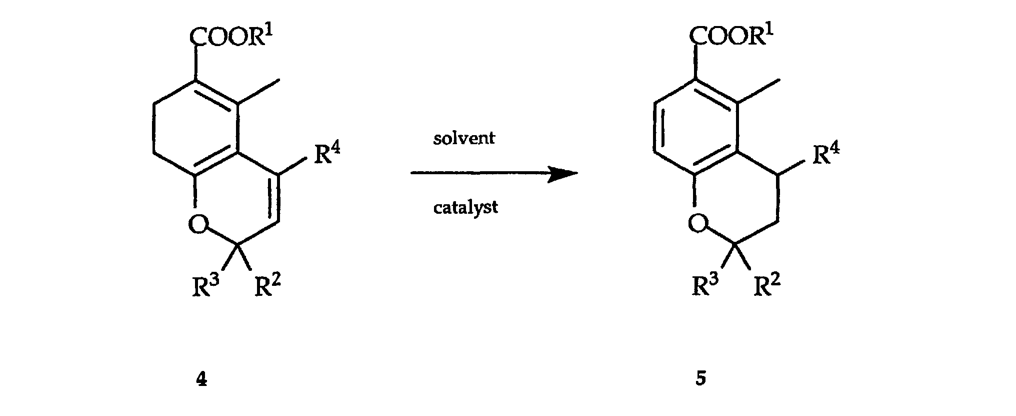 wherein
   R¹ is a straight or branched (C₁-C₅)alkyl, R², R³ and R⁴ are each independently a straight or branched (C₁-C₅)alkyl or a hydrogen atom, X is chloro, bromo, iodo, OSO₂R or OCOR, and R is alkyl or aryl.
   **or:**
b2. rearranging the propargyl ether (**3**) by application of heat with a suitable catalyst and solvent being present, to produce a chroman ester (**5**) 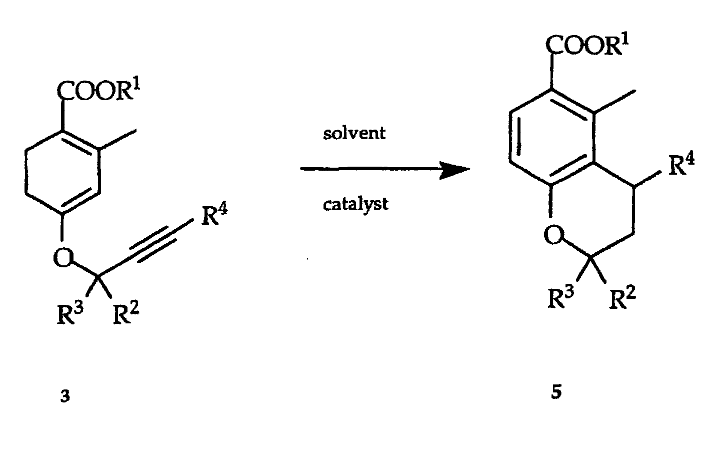 wherein
   R¹ is a straight or branched (C₁-C₅)alkyl,
   R², R³ and R⁴ are each independently a straight or branched (C₁-C₅)alkyl or a hydrogen atom,
   X is chloro, bromo, iodo, OSO₂R or OCOR, and
   R is alkyl or aryl.

The chroman esters (**5**) can be converted to the corresponding chroman acids (**6**) by methods, such as hydrolysis, well known to those of ordinary skill in the art:

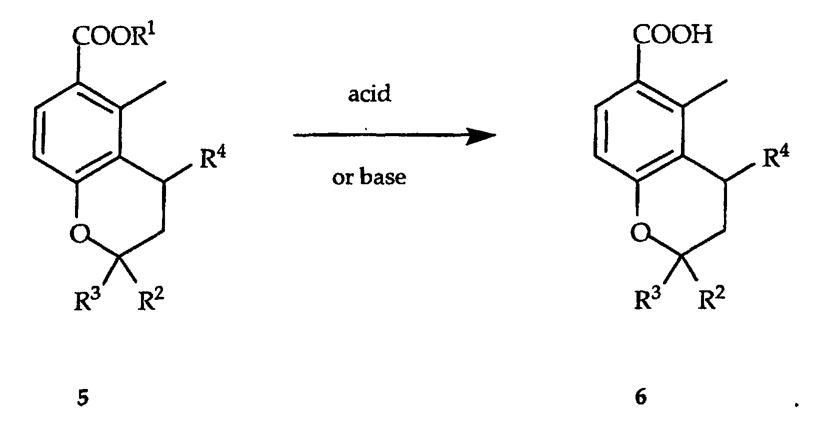

In the present invention, straight or branched (C₁-C₅)alkyl includes, for example, methyl, ethyl, *n*-propyl, isopropyl, *n*-butyl, isobutyl, *sec*-butyl, *tert*-butyl, *n*-amyl, isoamyl, 2-pentyl, 3-pentyl, 2-methyl-2-butyl, 3-methyl-2-butyl,
2,2-dimethylpropyl and the like.

Alkyl includes, for example, any of the straight or branched (C₁-C₅)alkyl groups as well as hexyl, octyl, decyl and the like.

Aryl includes, for example, phenyl, *p*-tolyl and the like.

In steps a, b and c of the described process, it is preferred that R¹ is methyl or ethyl, R², R³ and R⁴ are each independently methyl, ethyl or a hydrogen atom, and X is chloro, bromo or OSO₂R wherein R is *p*-tolyl. More preferred is when R², R³ and R⁴ are each a hydrogen atom and X is bromo.

Step a of the process of the present invention is run at a temperature of -50°C to 100°C, a pressure of 0.5 to 5 atmospheres, a time of 30 minutes to 2 days, an equivalent stoichiometry of the propargyl derivative (**2**) : the ester (**1**) being 1-3:1 and an equivalent stoichiometry of base : the ester (**1**) being 1-3:1. A preferred condition is when the reaction temperature is 0-100°C, the pressure is 1-2 atmospheres and the reaction time is 30 minutes - 12 hours. A more preferred condition is when the reaction temperature is 0-25°C, the reaction time is about 2 hours, the reaction pressure is about 1 atmosphere, and the equivalent stoichiometry of base : the ester (**1**) is about 1.2:1.

The solvent employed in step a can be a polar aprotic or protic solvent such as dimethylsulfoxide (DMSO), dimethylformamide (DMF), tetrahydrofuran (THF) or dioxane, or a polar protic solvent such as methanol, ethanol or *tert*-butyl alcohol. Preferred solvents include THF, DMSO, dioxane or alcohol. More preferred are THF or ethanol.

The base employed in step a can be R^{a}OM or (R^{a})₂NM wherein R^{a} is a straight chain or branched (C₁-C₄)alkyl and M is Na, Li or K; a hydride, for example NaH or KH; a carbonate, for example Li₂CO₃, Na₂CO₃ or K₂CO₃; or a hydroxide, for example LiOH, NaOH or KOH. Preferred bases include R^{a}OM or (R^{a})₂NM wherein R^{a} = C₁-C₄ straight chain or branched alkyl; or NaH. Most preferred bases are NaH or lithium diisopropylamide (LDA).

The ester (**1**) may be added to the base followed by the addition of the propargyl derivative (**2**) or the base may be added to the ester (**1**) followed by the addition of the propargyl derivative (**2**).

Purification of the intermediate (**3**), if desired, may be accomplished by short-path distillation or chromatography on silica gel or Florisil using methods well known to those of ordinary skill in the art.

Step b1 of the process of the present invention is run at a temperature of 25°C to 250°C, a pressure of 0.5 to 1000 atmospheres, a time of 30 minutes to 3 days, and an equivalent stoichiometry of the catalyst: the propargyl ether (**3**) being 0-10:1. A preferred condition is when the reaction temperature is 25-150°C, the pressure is 1-2 atmospheres and the reaction time is 2-24 hours. A more preferred condition is when the reaction temperature is about 60°C, the reaction time is about 4 hours and the reaction pressure is about 1 atmosphere.

The solvent optionally employed in step b1 is a moderate to high boiling solvent which is neutral, acidic or basic in nature and which is protic or aprotic in nature. Suitable solvents include, but are not limited to, chlorobenzene, toluene, xylene, decalin, diglyme, N,N-diethylaniline, pyridine, DMF,
N-methylpyrrolidine, quinoline, chloroform, straight or branched chain (C₁-C₅)alcohols, acetic acid, trichloroacetic acid, trifluoroacetic acid, 1,3-dimethyl-2-imidazolidinone (DMEU) and dimethylpropylidine urea (DMPU). Preferred solvents include N-methylpyrrolidine, N,N-diethylaniline or trifluoroacetic acid. Trifluoroacetic acid is more preferred.

Catalysts optionally used in step b1 include, but are not limited to, silver(I) salts such as AgOC(O)CF₃, mercury salts such as Hg(II)OC(O)CF₃, rhodium(I) complexes such as Rh₂Cl₂(CO)₄, Pt(0) complexes such as Pt[P(C₆H₅)₃]₂, Pd(0) complexes such as Pd[P(C₆H₅)₃]₄, Pd(II) complexes such as Pd[P(C₆H₅)₃]₂Cl₂ or Pd[(CH₃)₂NCH₂CH₂N(CH₃)₂]Cl₂, BF₃, BCl₃, (R^{b})ₙAlCl₍₃₋ₙ₎ wherein R^{b} is (C₁-C₅)alkyl and n is 1-2, ZnCl₂, TiCl₄, CF₃COOH, H₂SO₄ or H₃PO₄. Preferred catalysts are CF₃COOH, AgOC(O)CF₃ or BCl₃. More preferred is AgOC(O)CF₃.

The order of addition of the reactants and catalyst is not important.

Purification of the cyclic ether (**4**), if desired, may be accomplished by short-path distillation or chromatography on silica gel or Florisil using methods well known to those of ordinary skill in the art.

Step c1 of the process of the present invention is run at a temperature of 0°C to 100°C, a pressure of 0.5 to 5 atmospheres, a time of 30 minutes to 5 hours and an equivalent stoichiometry of the base or acid : cyclic ether (**4**) being
0.2-200:1. A preferred condition is when the reaction temperature is 25-50°C, the pressure is 1-2 atmospheres and the reaction time is 30 minutes - 1 hour. A more preferred condition is when the reaction temperature is about 25°C, the reaction time is about 30 minutes and the reaction pressure is about 1 atmosphere.

The solvent employed in step c1 using basic conditions can be a polar aprotic solvent such as DMSO, DMF, THF or dioxane, or a polar protic solvent such as methanol, ethanol or *tert*-butyl alcohol. Using acidic conditions the solvent can be a straight chain or branched (C₁-C₅)alcohol, water, THF or mixtures thereof, acetic acid, trifluoroacetic acid or trichloroacetic acid. Preferred solvents include acetic acid, trifluoroacetic acid, trichloroacetic acid or alcohol. More preferred is trifluoroacetic acid.

The catalyst employed in step c1, when a base, can be (R^{a})OM or (R^{a})₂NM wherein R^{a} is a straight chain or branched (C₁-C₄)alkyl and M is Na, Li or K; a hydride, for example NaH or KH; or a hydroxide, for example LiOH, NaOH or KOH, or when an acid, can be sulfuric acid, phosphoric acid, hydrochloric acid, hydrobromic acid, nitric acid, trifluoroacetic acid or acidic alumina. An acid is generally more preferred than a base. Preferred acids include sulfuric acid, phosphoric acid, hydrochloric acid, hydrobromic acid or trifluoroacetic acid. More preferred are sulfuric acid or trifluoroacetic acid.

The order of addition of the reactants and catalyst is not important.

Purification of the chroman ester (**5**), if desired, may be accomplished by short-path distillation or chromatography on silica gel or Florisil using methods well known to those of ordinary skill in the art.

In a preferred embodiment of this process, utilizing steep b2, the propargyl ether (**3**), wherein
R¹ is a straight or branched (C₁-C₅)alkyl and
R², R³ and R⁴ are each independently a straight or branched (C₁-C₅)alkyl or a hydrogen atom, can be converted directly to the chroman ester (**5**) under the reaction conditions of step b when a Lewis or Brönsted Acid catalyst is employed in that step. Examples of suitable catalysts in this regard include, but are not limited to, AgOC(O)CF₃, BF₃, BCl₃, (R^{b})ₙAlCl₍₃₋ₙ₎ wherein R^{b} is (C₁-C₅)alkyl and n is 1-2, CF₃COOH, H₂SO₄ or H₃PO₄. Preferred catalysts are CF₃COOH, AgOC(O)CF₃, H₂SO₄ or H₃PO₄. More preferred is AgOC(O)CF₃. Suitable solvents include, but are not limited to, acetic acid, trichloroacetic acid, trifluoroacetic acid, straight or branched chain (C₁-C₅)alcohols, THF, water or mixtures thereof. Preferred solvents are trifluoroacetic acid, acetic acid and trichloroacetic acid. A more preferred solvent is trifluoroacetic acid.

In another embodiment of this invention, the propargyl ethers (**3**) and the cyclic ether type compounds (**4**), wherein
R¹ is a straight or branched (C₁-C₅)alkyl and
R², R³ and R⁴ are each independently a straight or branched (C₁-C₅)alkyl or a hydrogen atom,
produced by the process of the present invention are new.

It should be understood that the instant specification is set forth by way of illustration and not limitation.

## Claims

1. A process to produce chroman esters which comprises the steps of
a. reacting a Hagemann's ester (**1**) with a propargyl derivative (**2**) in the presence of a base and a solvent to produce a propargyl ether (**3**) 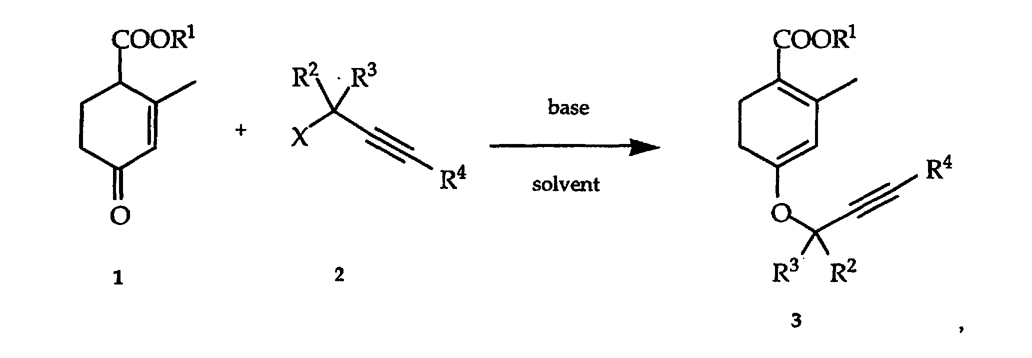 Wherein
step a of the process is run at a temperature of -50°C to 100°C, a pressure of 0.5 to 5 atmospheres, a time of 30 minutes to 2 days, an equivalent stoichiometry of the propargyl derivative (**2**): the ester (**1**) being
1-3:1 and an equivalent stoichiometry of base : the ester (**1**) being 1-3:1.
Wherein
the solvent employed in step a is a polar aprotic or protic solvent.
followed by either:
b1. rearranging the propargyl ether (**3**) by application of heat, optionally with a suitable catalyst and a solvent being present, to produce a cyclic ether type compound (**4**) 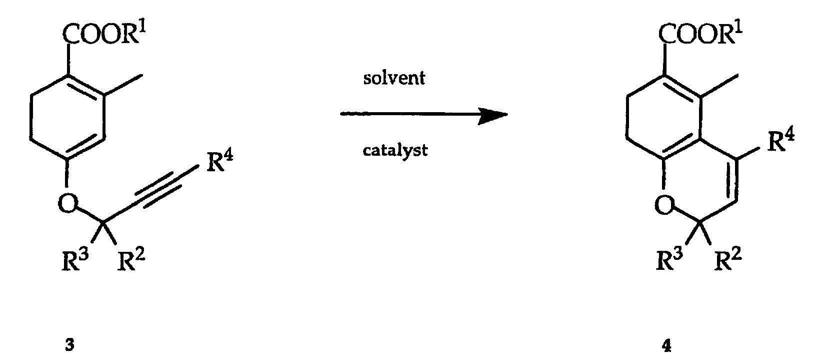 Wherein
step b1 of the process is run at a temperature of 25°C to 250°C, a pressure of 0.5 to 1000 atmospheres, a time of 30 minutes to 3 days, and an equivalent stoichiometry of the catalyst: the propargyl ether (**3**) being 0-10:1.
Wherein
the solvent optionally employed in b 1 is a moderate to high boiling solvent which is neutral, acidic or basic in nature and which is protic or aprotic in nature.
Wherein
the catalyst optionally employed in step b1 is a silver(I) salt, a mercury salt, a rhodium(I) complex, a Pt(0) complex, a Pd(0) complex, a Pd(II) complex, BF₃, BCl₃, (R^{b})ₙAlCl₍₃₋ₙ₎ wherein R^{b} is (C₁-C₅)alkyl and n is 1-2, ZnCl₂, TiCl₄, CF₃COOH, H₂SO₄ or H₃PO₄.
and
c1. isomerizing the cyclic ether type compound (**4**) with a catalyst in a suitable solvent to produce a chroman ester (**5**) 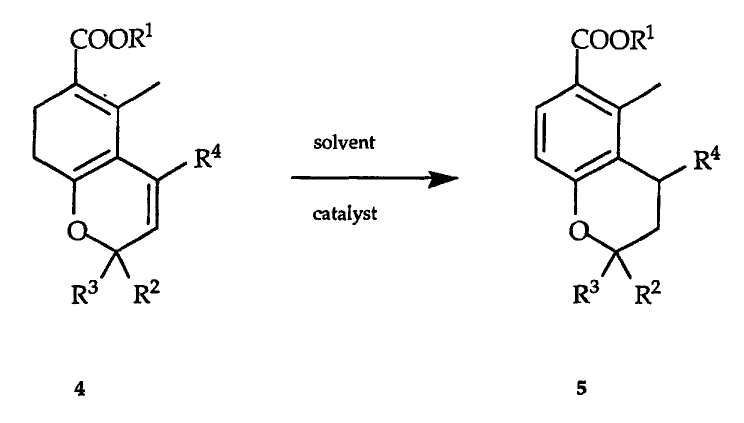 wherein
R¹ is a straight or branched (C₁-C₅)alkyl, R², R³ and R⁴ are each independently a straight or branched (C₁-C₅)alkyl or a hydrogen atom, X is chloro, bromo, iodo, OSO₂R or OCOR, and R is alkyl or aryl.
Wherein
step c1 of the process is run at a temperature of 0°C to 100°C, a pressure of 0.5 to 5 atmospheres, a time of 30 minutes to 5 hours and an equivalent stoichiometry of the base or acid : cyclic ether (**4**) being 0.2-200:1.
Wherein
the solvent employed in step c1 using basic conditions is a polar aprotic solvent or a polar protic solvent or using acidic conditions the solvent is a straight chain or branched (C₁-C₅)alcohol, water, THF or mixtures thereof, acetic acid, trifluoroacetic acid or trichloroacetic acid.
Wherein
the catalyst employed in step c1, when a base, is (R^{a})OM or (R^{a})₂NM wherein R^{a} is a straight chain or branched (C₁-C₄)alkyl and M is Na, Li or K; a hydride; or a hydroxide; or when an acid, is sulfuric acid, phosphoric acid, hydrochloric acid, hydrobromic acid, nitric acid, trifluoroacetic acid or acidic alumina.
**or:**
b2 rearranging the propargyl ether (**3**) by application of heat with a suitable catalyst and solvent being present, to produce a chroman ester (**5**) 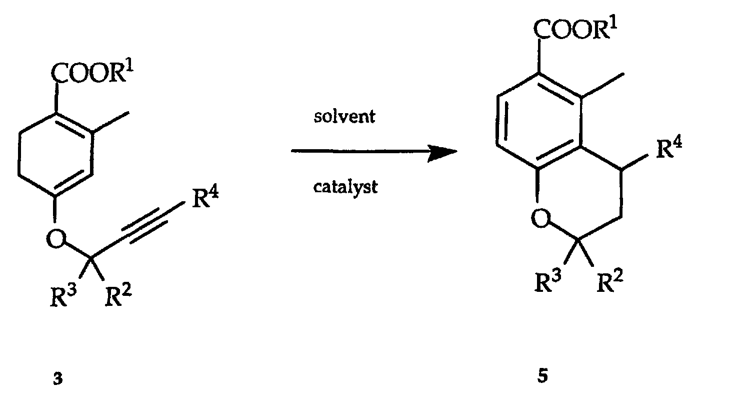 wherein
R¹ is a straight or branched (C₁-C₅)alkyl, R², R³ and R⁴ are each independently a straight or branched (C₁-C₅)alkyl or a hydrogen atom, X is chloro, bromo, iodo, OSO₂R or OCOR, and R is alkyl or aryl.
Wherein
the solvent optionally employed in step b2 is acetic acid, trichloroacetic acid, trifluoroacetic acid, straight or branched chain (C₁-C₅)alcohols, THF, water or mixtures thereof.
Wherein
the catalyst optionally employed in step b2 is selected from a silver(I) salt, BF₃, BCl₃, (R^{b})ₙAlCl₍₃₋ₙ₎ wherein R^{b} is (C₁-C₅)alkyl and n is 1-2, CF₃COOH, H₂SO₄ or H₃PO₄.

2. The process of claim 1 wherein
R¹ is methyl or ethyl,
R², R³ and R⁴ are each independently methyl, ethyl or a hydrogen atom,
X is chloro, bromo or OSO₂R, and
R is *p*-tolyl.

3. The process of claim 2 wherein R², R³ and R⁴ are each a hydrogen atom.

4. The process of claims 2 or 3 wherein X is bromo.

5. The process of claim 1 wherein the catalyst, in step b1 is selected from CF₃COOH, AgOC(O)CF₃ or BCl₃.

6. The process of claim 1 wherein the catalyst, in step bl is AgOC(O)CF₃.

7. A compound of the formula 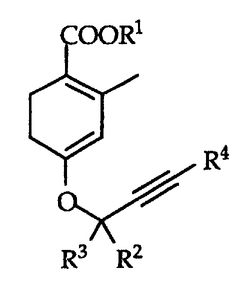 wherein
R¹ is a straight or branched (C₁-C₅)alkyl and
R², R³ and R⁴ are each independently a straight or branched (C₁-C₅)alkyl or a hydrogen atom.

8. The compound of claim 7 wherein R¹ is methyl or ethyl and R², R³ and R⁴ are each independently methyl, ethyl or a hydrogen atom.

9. The compound of claim 8 wherein R², R³ and R⁴ are each a hydrogen atom.

## Patentansprüche

1. Verfahren zum Herstellen eines Chromanesters, umfassend die Schritte
a. Umsetzen eines Hagemann-Esters (1) mit einem Propargylderivat (2) in der Gegenwart einer Base und eines Lösungsmittels, um einen Propargylether (3) zu bilden 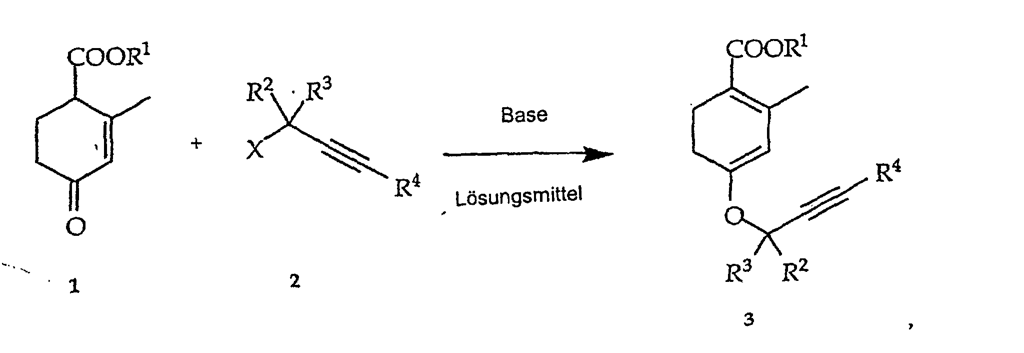 worin
Schritt a des Verfahrens bei einer Temperatur von -50 °C bis 100 °C, einem Druck von 0,5 bis 5 Atmosphären, in einer Zeit von 30 Minuten bis 2 Tagen durchgeführt wird, wobei eine Äquivalentstöchiometrie von Propargylderivat (2): Ester (1) 1-3:1 und eine Äquivalentstöchiometrie von Base : Ester (1) 1-3:1 ist;
worin
das in Schritt a verwendete Lösungsmittel ein polares aprotisches oder protisches Lösungsmittel ist,
gefolgt durch entweder:
b1. Umlagern des Propargylethers (3) durch Anwendung von Hitze, wobei gegebenenfalls ein geeigneter Katalysator und ein Lösungsmittel vorliegen, um eine Etherverbindung des cyclischen Typs (4) zu erzeugen 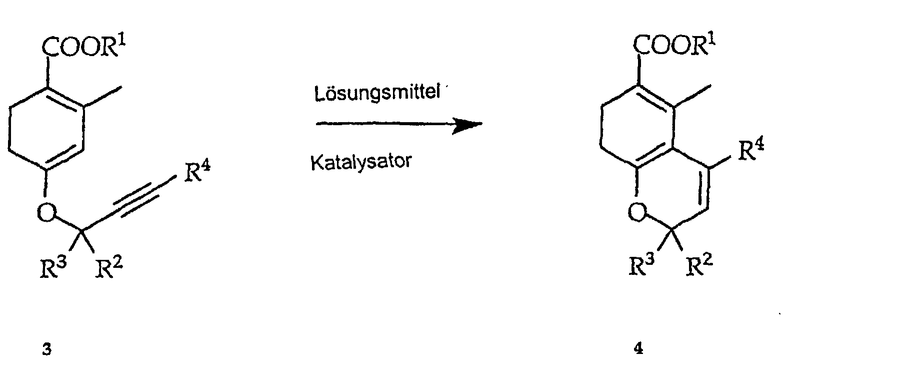 worin
Schritt b1 des Verfahrens bei einer Temperatur von 25 °C bis 250 °C, einem Druck von 0,5 bis 1000 Atmosphären, in einer Zeit von 30 Minuten bis 3 Tagen durchgeführt wird und eine Äquivalentstöchiometrie von Katalysator : Propargylether (3) 0 bis 10:1 ist;
worin
das Lösungsmittel, das optional in b1 verwendet wird, ein moderat bis hoch siedendes Lösungsmittel ist, das neutral, sauer oder basisch ist und welches protisch oder aprotisch ist;
worin
der optional in Schritt b1 verwendete Katalysator ein Silber(I)-Salz, ein Quecksilbersalz, ein Rhodium(I)-Komplex, ein Pt(O)-Komplex, ein Pd(O)-Komplex, ein Pd(II)-Komplex, BF₃, BCl₃, (R^{b})ₙAlCl₍₃₋ₙ₎ ist, worin R^{b} (C₁-C₅)-Alkyl ist und n 1 bis 2 ist, ZnCl₂, TiCl₄, CF₃COOH, H₂SO₄ oder H₃PO₄ ist;
und
c1: Isomerisieren der Verbindung des cyclischen Ethertyps (4) mit einem Katalysator in einem geeigneten Lösungsmittel, um einen Chromanester (5) zu erzeugen 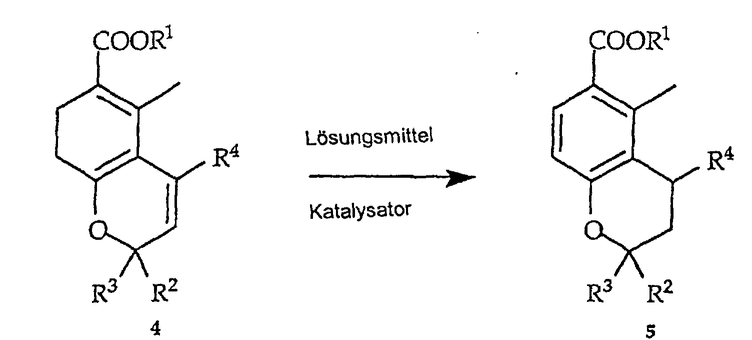 worin
R¹ ein geradkettiges oder verzweigtes (C₁-C₅)-Alkyl ist, R². R³ und R⁴ jeweils unabhängig ein geradkettiges oder verzweigtes (C₁-C₅)-Alkyl oder ein Wasserstoffatom sind, X Chlor, Brom, lod, OSO₂R oder OCOR ist und R Alkyl oder Aryl ist;
worin
Schritt c1 des Verfahrens bei einer Temperatur von 0 °C bis 100 °C, einem Druck von 0,5 bis 5 Atmosphären, in einer Zeit von 30 Minuten bis 5 Stunden durchgeführt wird und wobei eine Äquivalentstöchiometrie von Base oder Säure : cyclischer Ether (4) 0,2 bis 200:1 ist;
worin
das in Schritt c1 unter Verwendung basischer Bedingungen verwendete Lösungsmittel ein polares aprotisches Lösungsmittel oder ein polares protisches Lösungsmittel ist oder wobei unter Verwendung saurer Bedingungen das Lösungsmittel ein geradkettiger oder verzweigter (C₁-C₅)-Alkohol, Wasser, THF oder Gemische davon, Essigsäure, Trifluoressigsäure oder Trichloressigsäure ist;
worin
der in Schritt c1 verwendete Katalysator, wenn er eine Base ist, (R^{a})OM oder (R^{a})₂NM, worin R^{a} ein geradkettiges oder verzweigtes (C₁-C₄)-Alkyl ist und M Na, Li oder K ist; ein Hydrid; oder ein Hydroxid ist; oder wenn er eine Säure ist, Schwefelsäure, Phosphorsäure, Chlorwasserstoffsäure, Bromwasserstoffsäure, Salpetersäure, Trifluoressigsäure oder saures Aluminiumoxid ist;
oder:
b2: Umlagern des Propargylethers (3) durch Anwendung von Hitze mit einem geeigneten Katalysator und bei Vorliegen von Lösungsmittel, um einen Chromanester (5) 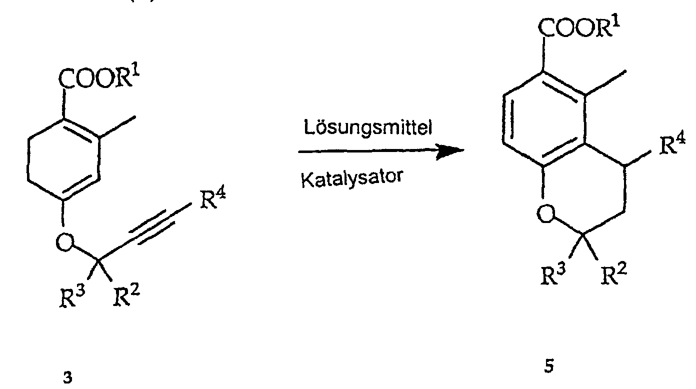 zu bilden,
worin
R¹ ein geradkettiges oder verzweigtes (C₁-C₅)-Alkyl ist, R², R³ und R⁴ jeweils unabhängig ein geradkettiges oder verzweigtes (C₁-C₅)-Alkyl oder ein Wasserstoffatom sind, X Chlor, Brom, lod, OSO₂R oder OCOR ist und R Alkyl oder Aryl ist;
worin
das optional in Schritt b2 verwendete Lösungsmittel Essigsäure, Trichloressigsäure, Trifluoressigsäure, geradkettige oder verzweigtkettige (C₁-C₅)-Alkohole, THF, Wasser oder Gemische davon ist;
worin
der Katalysator, der optional in Schritt b2 verwendet wird, ausgewählt wird aus einem Silber(I)-Salz, BF₃, BCl₃, (R^{b})ₙAlCl₍₃₋ₙ₎, worin R^{b} (C₁-C₅)-Alkyl ist und n 1 bis 2 ist, CF₃COOH, H₂SO₄ oder H₃PO₄.

2. Verfahren nach Anspruch 1, worin
R¹ Methyl oder Ethyl ist,
R², R³ und R⁴ jeweils unabhängig Methyl, Ethyl oder Wasserstoffatome sind,
X Chlor, Brom oder OSO₂R ist und
R p-Tolyl ist.

3. Verfahren nach Anspruch 2, worin R², R³ und R⁴ jeweils ein Wasserstoffatom sind.

4. Verfahren nach Anspruch 2 oder 3, worin X Brom ist.

5. Verfahren nach Anspruch 1, worin der Katalysator von Schritt b1 ausgewählt wird aus CF₃COOH, AgOC(O)CF₃ oder BCl₃.

6. Verfahren nach Anspruch 1, worin der Katalysator in Schritt b1 AgOC(O)CF₃ ist.

7. Verbindung der Formel 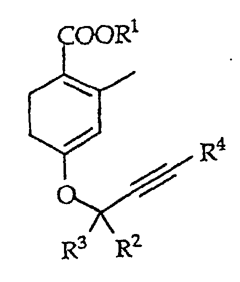 worin
R¹ ein geradkettiges oder verzweigtes (C₁-C₅)-Alkyl ist und
R², R³ und R⁴ jeweils unabhängig ein geradkettiges oder verzweigtes (C₁-C₅)-Alkyl oder ein Wasserstoffatom sind.

8. Verbindung nach Anspruch 7, worin R¹ Methyl oder Ethyl ist und R², R³ und R⁴ jeweils unabhängig Methyl, Ethyl oder ein Wasserstoffatom sind.

9. Verbindung nach Anspruch 8, worin R², R³ und R⁴ jeweils ein Wasserstoffatom sind.

## Revendications

1. Procédé de préparation d'esters dérivés du chromane, qui comprend les étapes consistant à :
a) faire réagir un ester de Hagemann (1) avec un dérivé propargylique (2), en présence d'une base et d'un solvant, pour produire un éther propargylique (3) 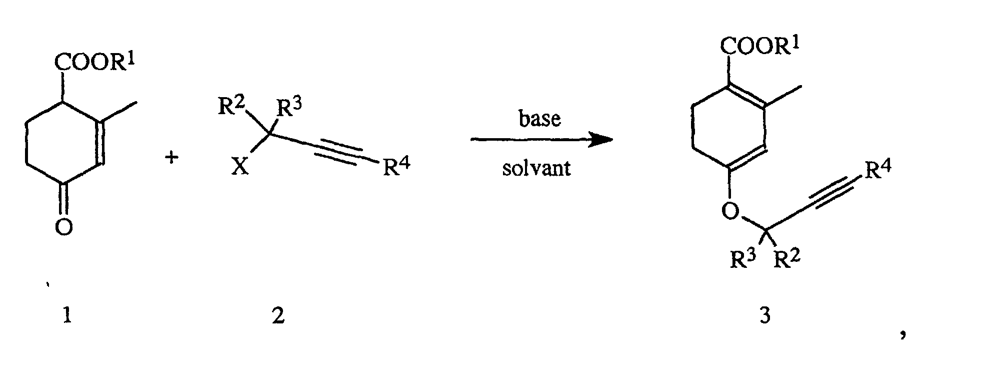 l'étape a) du procédé étant réalisée à une température de -50°C à 100°C, sous une pression de 0,5 à 5 atmosphères, pendant une durée de 30 minutes à 2 jours, et avec un rapport des équivalents du dérivé propargylique (2) aux équivalents de l'ester (1) de 1-3 :1 et un rapport des équivalents de la base aux équivalents de l'ester (1) de 1-3:1,
le solvant employé dans l'étape a) étant un solvant polaire, protique ou aprotique,
puis :
b1) réarranger l'éther propargylique (3) par application de la chaleur, éventuellement en présence d'un catalyseur approprié et d'un solvant, pour produire un composé (4) du type éther cyclique 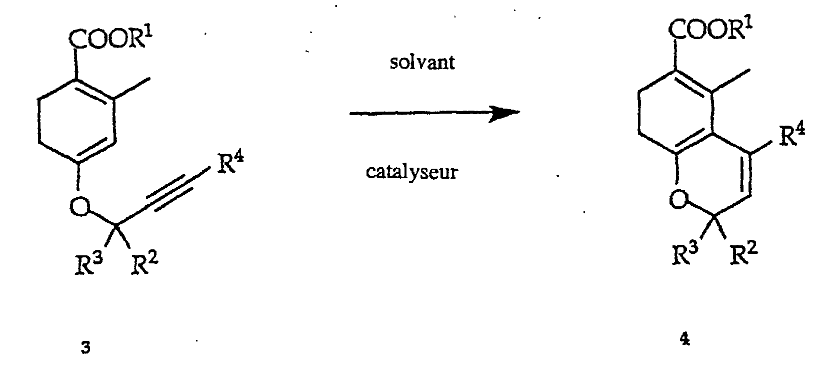 l'étape b1) du procédé étant réalisée à une température de 25°C à 250°C, sous une pression de 0,5 à 1000 atmosphères, pendant une durée de 30 minutes à 3 jours, et avec un rapport des équivalents du catalyseur aux équivalents de l'éther propargylique (3) de 0-10:1,
le solvant éventuellement employé dans l'étape b1) étant un solvant à point d'ébullition modéré à élevé, qui est de nature neutre, acide ou basique et qui est de nature protique ou aprotique,
et le catalyseur éventuellement employé dans l'étape b1) étant un sel d'argent (I), un sel de mercure, un complexe du rhodium (I), un complexe de Pt(0), un complexe de Pd(0), un complexe de Pd(II), BF₃, BCl₃, (R^{b})ₙAlCl₍₃₋ₙ₎ où R^{b} représente un groupe alkyle en C₁-C₅ et n est égal à 1 ou 2, ZnCl₂, TiCl₄, CF₃COOH, H₂SO₄ ou H₃PO₄,
et
c1) isomériser le composé (4) du type éther cyclique, en présence d'un catalyseur, dans un solvant approprié, pour produire un ester (5) dérivé du chromane 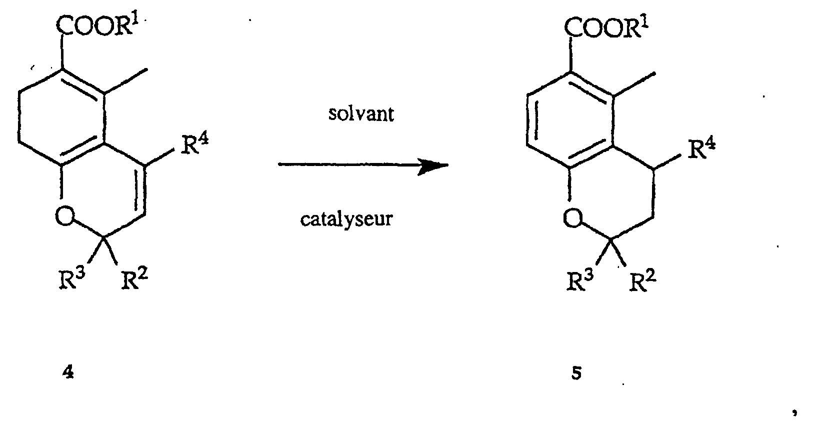 l'étape c1) du procédé étant réalisée à une température de 0°C à 100°C, sous une pression de 0,5 à 5 atmosphères, pendant une durée de 30 minutes à 5 heures, et avec un rapport des équivalents de la base ou de l'acide aux équivalents de l'éther cyclique (4) de 0,2-200:1,
le solvant employé dans l'étape c1) étant, lorsqu'on utilise des conditions basiques, un solvant polaire aprotique ou un solvant polaire protique, ou étant, lorsqu'on utilise des conditions acides, un alcool en C₁à C₅, à chaîne droite ou ramifiée, l'eau, le THF ou un mélange des produits précédents, l'acide acétique, l'acide trifluoroacétique ou l'acide trichloroacétique,
et le catalyseur employé dans l'étape c1) étant, lorsqu'il s'agit d'une base, (R^{a})OM ou (R^{a})₂NM, où Rₐ représente un groupe alkyle en C₁ à C₄, à chaîne droite ou ramifiée, et M représente Na, Li ou K, un hydrure ou un hydroxyde, ou bien, lorsqu'il s'agit d'un acide, l'acide sulfurique, l'acide phosphorique, l'acide chlorhydrique, l'acide bromhydrique, l'acide nitrique, l'acide trifluoroacétique ou l'alumine acide,
R¹ représentant dans les formules précédentes un groupe alkyle en C₁ à C₅, à chaîne droite ou ramifiée, R², R³ et R⁴ représentant chacun indépendamment un atome d'hydrogène ou un groupe alkyle en C₁ à C₅, à chaîne droite ou ramifiée, X représentant un atome de chlore, de brome ou d'iode, ou un groupe OSO₂R ou OCOR, R désignant un groupe alkyle ou aryle,
ou bien :
b2) réarranger l'éther propargylique (3) par application de la chaleur, en présence d'un catalyseur approprié et d'un solvant, pour produire un ester (5) dérivé du chromane 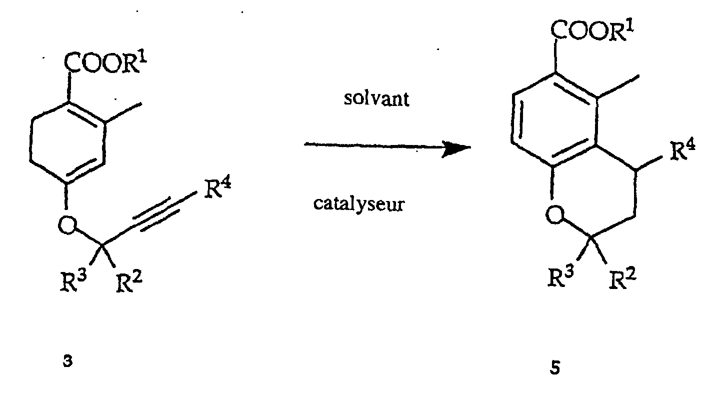 le solvant employé dans l'étape b2) étant l'acide acétique, l'acide trichloroacétique, l'acide trifluoroacétique, un alcool en C₁ à C₅ à chaîne droite ou ramifiée, le THF, l'eau ou un mélange de ces produits,
et le catalyseur employé dans l'étape b2) étant un sel d'argent (I), BF₃, BCl₃, (R^{b})ₙAlCl₍₃₋ₙ₎, où R^{b} représente un groupe alkyle en C₁ à C₅ et n est égal à 1 ou 2, CF₃COOH, H₂SO₄ ou H₃PO₄,
R¹ représentant dans les formules précédentes un groupe alkyle en C₁ à C₅ à chaîne droite ou ramifiée, R², R³ et R⁴ représentant chacun indépendamment un atome d'hydrogène ou un groupe alkyle en C₁ à C₅ à chaîne droite ou ramifiée, et X représentant un atome de chlore, de brome ou d'iode, ou un groupe OSO₂R ou OCOR, R désignant un groupe alkyle ou aryle.

2. Procédé selon la revendication 1, dans lequel R¹ représente un groupe méthyle ou éthyle, R², R³ et R⁴ représentent chacun indépendamment un groupe méthyle ou éthyle ou un atome d'hydrogène, X représente un atome de chlore ou de brome ou le groupe OSO₂R et R représente le groupe *p*-tolyle.

3. Procédé selon la revendication 2, dans lequel R², R³ et R⁴ représentent chacun un atome d'hydrogène.

4. Procédé selon la revendication 2 ou 3, dans lequel X représente un atome de brome.

5. Procédé selon la revendication 1, dans lequel le catalyseur utilisé dans l'étape b1) est CF₃COOH, AgOC(O)CF₃ ou BCl₃.

6. Procédé selon la revendication 1, dans lequel le catalyseur utilisé dans l'étape b1) est AgOC(O)CF₃.

7. Composé de formule : 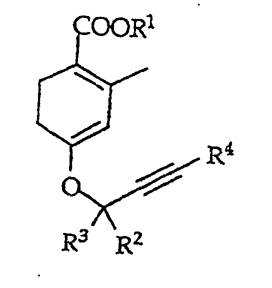 dans laquelle R¹ représente un groupe alkyle en C₁ à C₅, à chaîne droite ou ramifiée, et R², R³ et R⁴ représentent chacun indépendamment un atome d'hydrogène ou un groupe alkyle en C₁ à C₅, à chaîne droite ou ramifiée.

8. Composé selon la revendication 7, pour lequel R¹ représente un groupe méthyle ou éthyle et R², R³ et R⁴ représentent chacun indépendamment un atome d'hydrogène ou un groupe méthyle ou éthyle.

9. Composé selon la revendication 8, pour lequel R², R³ et R⁴ représentent chacun un atome d'hydrogène.
